# EUROPÄISCHE PATENTANMELDUNG

(11) **EP 1 127 506 A2**
(43) Veröffentlichungstag der Anmeldung: **29.08.2001**
(21) Anmeldenummer: 01104279.3
(22) Anmeldetag: 22.02.2001
(51) Int. Cl.: A43B 7/36

(54) **Antistatische Fussbekleidung zur Abführung statischer Aufladungen**

(30) Priorität: 24.02.2000 DE 20003473 U
(71) Anmelder: Stucke, Michael, 30449 Hannover (DE)
(72) Erfinder: Stucke, Michael, 30449 Hannover (DE)

(57) **Zusammenfassung**

Die Erfindung betrifft eine antistatische Fußbekleidung zur Abführung statischer Aufladungen, die dadurch gekennzeichnet ist, daß die Schuhsohle (2) aus einem Material beliebiger elektrischer Leitfähigkeit, wie z. B. Leder, Gummi, Kunststoff usw., besteht, und die Aufladung des Körpers großflächig im Fußsohlenbereich abgeführt wird.

## Beschreibung

Die Erfindung betrifft eine antistatische Fußbekleidung zur Abführung statischer Aufladungen.

Es ist aus der Physik bekannt, daß sich der menschliche Körper unter bestimmten Bedingungen extrem stark elektrisch aufladen kann. Bei Berührung des Körpers mit einer leitfähigen Fläche bzw. einem Gegenstand können große Funken überspringen. Dieser Funke erzeugt auf der einen Seite einen starken Schmerzreiz und kann die Konzentrationsfähigkeit bzw. das Wohlbefinden der jeweiligen Person stark negativ beeinflussen. Andererseits kann es in Bereichen, die explosionsgeschützt betrieben werden müssen, zu unkontrollierten Entladungen kommen (Lösemittelexplosion, Staubentzündung usw.), welche extreme Schäden verursachen.

Es ist bekannt, daß z. B. in der Praxis - um ein direktes Überspringen des Funkens von einem Finger auf ein Metallteil zu vermeiden - ein zusätzliches Metallteil in die Hand genommen wird. Im Regelfall wird hier ein stiftförmiger Gegenstand benutzt, den die Person zur Ableitung der elektrostatischen Aufladung benutzt. Voraussetzung ist jedoch, daß die jeweilige Person Kenntnis vom Status ihres Aufladungszustandes besitzt.

Bekannt sind z. B. Lösungen für den Wohnbereich, wo sehr oft zur Ableitung elektrostatischer Aufladungen von Personen antistatische Teppiche benutzt werden, welche mitunter durch chemische Imprägnierungen bzw. den Einsatz leitfähiger Fasern verstärkt werden. Die Funktion dieser Leitflächen ist jedoch sehr oft von der Höhe der Raumluftfeuchtigkeit sowie vom Verschmutzungsgrad abhängig.

Bei anderen bekannten Lösungen im Schuhbereich werden z.B. Schuhsohlen und Absätze aus antistatischen Materialien hergestellt. Diese Werkstoffe bieten sehr oft keinen Tragekomfort und ermöglichen kaum die notwendige Abfuhr der Feuchtigkeit im Fußbereich.

Beschrieben wurde bereits auch eine Schuhvariante mit einer Entladungsvorrichtung für elektrostatische Potentiale. Hierbei werden 2 Elektroden benutzt, wobei die Innenelektrode mit der Außenelektrode über eine elektrische Verbindung gekoppelt ist. Bei dieser Lösung sind die Elektroden vorzugsweise im Bereich des Absatzes angeordnet und die galvanische Verbindung zur Außenelektrode federelastisch ausgeführt, um die Fußbewegungen zu überbrücken. Bei dieser Lösung ist der Aufbau des Systems kompliziert und bietet darüber hinaus nur eine partielle Ableitungsmöglichkeit im Fersenbereich, so daß eine sichere Entladung nicht gewährleistet ist.

Bei anderen bekannten Lösungen werden durch die Schuhsohle elektrisch leitende Körper, z. B. Metallstifte, hindurchgeführt. Bei diesen Maßnahmen sind im Hinblick auf preiswerte Herstellungstechniken keine akzeptablen Lösungen sichtbar. Darüber hinaus führen die bis zum Fußsohlenbereich durchgehenden Stifte zu einer erkennbaren Minderung des Tragekomforts.

Der Erfindung liegt daher die Aufgabe zugrunde, zur Verhinderung gefährlicher elektrostatischer Aufladung von Personen eine antistatisch wirkende Fußbekleidung zu schaffen, welche bei einfacher Herstellung auch unter extremen Bedingungen - bei gleichbleibendem Tragekomfort - elektrostatische Potentiale ableitet.

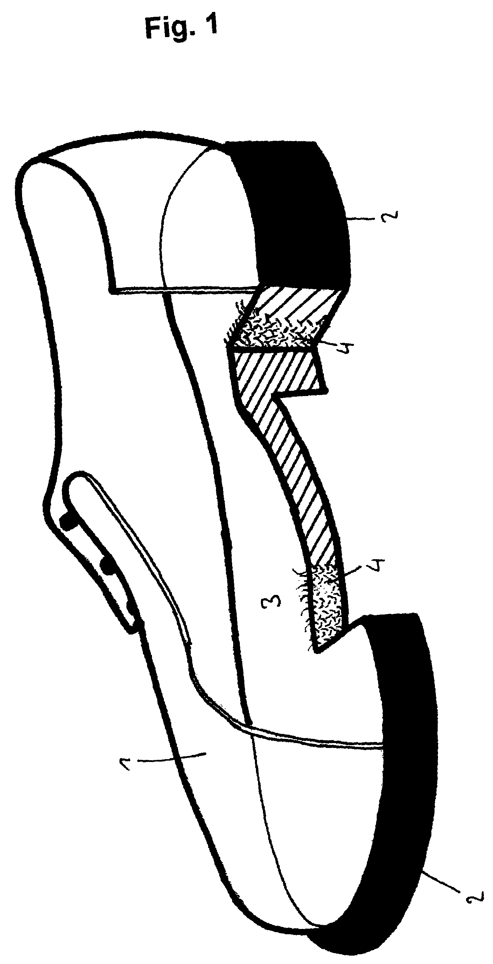

Eine mögliche Ausführungsform der Erfindung ist in Fig. 1 dargestellt. Die antistatische Fußbekleidung (1), z. B. Schuh, besteht im Regelfall im Oberteil aus Leder, während die Sohle (2) aus den üblichen Materialien wie Gummi, Kunststoff, Leder usw. hergestellt ist. Die leitende dünne Einlegesohle (3) besteht im Regelfall aus Aluminium und ist elektrisch leitend über das Leitergewebe (4) mit dem Bodenbereich unterhalb der Sohle (2) verbunden.

Die Aufgabe der Erfindung wird durch eine antistatische Fußbekleidung zur Abführung statischer Aufladungen gelöst, wobei die Schuhsohle aus einem Material beliebiger Leitfähigkeit wie z. B. Leder, Gummi, Kunststoff usw. besteht und die Aufladung des Körpers großflächig im Fußsohlenbereich an den darunterliegenden Boden abgeführt wird. Bei einer erfindungsgemäßen Variante ist in die Sohle ein leitfähiges Material eingebettet, das sich natürlich im vorderen wie auch im hinteren Bereich (Absatz) befinden kann. Als leitfähiges Material wird vorzugsweise eine flache Litze aus Kupferfasern benutzt. Alternativ können bei anderen erfindungsgemäßen Lösungen auch leitfähige Kohlenstoffasern oder Kunststoffasern benutzt werden.

Die Einlegesohle (3), welche als schuhinnere Basis der elektrischen Ableitung dient, ist als dünne Metallfolie ausgeführt, die im Regelfall von einer Abdecksohle geschützt wird. Die leitende Metallfolie kann zur Verbesserung der Feuchtigkeitsableitung im Fußbereich perforiert oder mit entsprechenden Durchbrüchen versehen werden.

### Bezugszeichenliste:

1) Schuh
2) Sohle
3) Einlegesohle
4) Leitergewebe

## Patentansprüche

1. Antistatische Fußbekleidung zur Abführung statischer Aufladungen,
**dadurch gekennzeichnet,**
daß die Schuhsohle (2) aus einem Material beliebiger elektrischer Leitfähigkeit, wie z. B. Leder, Gummi, Kunststoff usw., besteht, und die Aufladung des Körpers großflächig im Fußsohlenbereich abgeführt wird.

2. Antistatische Fußbekleidung zur Abführung statischer Aufladungen, nach Anspruch 1,
**dadurch gekennzeichnet,**
daß in die Sohle (2) leitfähiges Material eingebettet ist.

3. Antistatische Fußbekleidung zur Abführung statischer Aufladungen, nach den Ansprüchen 1 und 2,
**dadurch gekennzeichnet,**
daß in den Schuhabsatz leitfähiges Material eingebettet ist.

4. Antistatische Fußbekleidung zur Abführung statischer Aufladungen, nach einem oder mehreren der Ansprüche 1 - 3,
**dadurch gekennzeichnet,**
daß als leitfähiges Material Kupferfasern eingesetzt werden.

5. Antistatische Fußbekleidung zur Abführung statischer Aufladungen, nach einem oder mehreren der Ansprüche 1 - 4,
**dadurch gekennzeichnet,**
daß die Kupferfasern (4) als bandförmig geflochtenes Leiterkabel ausgeführt sind.

6. Antistatische Fußbekleidung zur Abführung statischer Aufladungen, nach einem oder mehreren der Ansprüche 1 - 5,
**dadurch gekennzeichnet,**
daß als leitfähiges Material Kohlenstoffasern eingebettet sind.

7. Antistatische Fußbekleidung zur Abführung statischer Aufladungen, nach einem oder mehreren der Ansprüche 1 - 6,
**dadurch gekennzeichnet,**
daß als leitfähiges Material elektrisch leitende Kunststoffasern eingebettet sind.

8. Antistatische Fußbekleidung zur Abführung statischer Aufladungen, nach einem oder mehreren der Ansprüche 1 - 7,
**dadurch gekennzeichnet,**
daß als schuhinnere Basis des elektrischen Ableitungssystems eine dünne Metallfolie in Form einer Einlegesohle (3) eingesetzt wird.

9. Antistatische Fußbekleidung zur Abführung statischer Aufladungen, nach einem oder mehreren der Ansprüche 1 - 8,
**dadurch gekennzeichnet,**
daß die Metallfolie zur Ableitung der Feuchtigkeit im Fußbereich perforiert oder mit Durchbrüchen versehen ist.

10. Antistatische Fußbekleidung zur Abführung statischer Aufladungen, nach einem oder mehreren der Ansprüche 1 - 9,
**dadurch gekennzeichnet,**
daß zur Verbesserung des Feuchtigkeitstransportes im Fußbereich über der Metallfolie eine Sammel- und Transportsohle vorgesehen ist.

11. Antistatische Fußbekleidung zur Abführung statischer Aufladungen, nach einem oder mehreren der Ansprüche 1 - 10,
**dadurch gekennzeichnet,**
daß das Schuhoberteil (1) aus Leder besteht.

12. Antistatische Fußbekleidung zur Abführung statischer Aufladungen, nach einem oder mehreren der Ansprüche 1 - 11,
**dadurch gekennzeichnet,**
daß die Einlegesohle (3) aus Aluminium elektrisch leitfähig mit dem Leitergewebe (4) verbunden ist.
